(19) Europäisches Patentamt
European Patent Office
Office européen des brevets

(11) Publication number: **0 047 536**
**B1**

(12) # EUROPEAN PATENT SPECIFICATION

(45) Date of publication of patent specification: **27.06.84**

(51) Int. Cl.³: **C 07 C 103/28**

(21) Application number: **81109790.6**

(22) Date of filing: **02.07.79**

(60) Publication number of the earlier application in accordance with Art. 76 EPC: **0007206**

(54) **Substituted propylamines.**

(30) Priority: **03.07.78 US 921668**

(43) Date of publication of application: **17.03.82 Bulletin 82/11**

(45) Publication of the grant of the patent: **27.06.84 Bulletin 84/26**

(84) Designated Contracting States: **DE GB IT NL SE**

(56) References cited: **FR - A - 1 555 583**

(73) Proprietor: **ELI LILLY AND COMPANY**
**307, East McCarty Street**
**Indianapolis Indiana 46285 (US)**

(72) Inventor: **Schmiegel, Klaus Kurt**
**4507, Staughton Drive**
**Indianapolis Indiana 46226 (US)**
Inventor: **Toomey, Richard Eugene**
**11425 Lakeshore Drive West**
**Carmel Indiana 46032 (US)**
Inventor: **Mills, Jack**
**deceased (US)**

(74) Representative: **Crowther, Terence Roger et al,**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 047 536 B1

**Description**

This invention relates to the compound 1,1-dimethyl-3-(4-aminocarbonylphenyl)propylamine.

Our co-pending application No. 79301282.4, publication number 0 007 206 relates to compounds of formula (I)

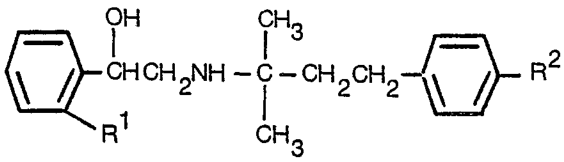

(I)

in which $R^1$ is hydrogen or fluorine and $R^2$ is hydroxy, aminocarbonyl, methylaminocarbonyl, dimethylaminocarbonyl, acetylamino or methanesulphonylamino, and the pharmaceutically acceptable acid addition salts thereof.

The compound of this invention is useful in the preparation of compounds of formula (I) and their salts in which $R^2$ is aminocarbonyl.

This invention therefore provides a compound of formula

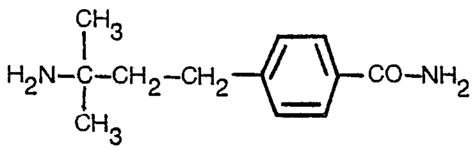

The compound of the invention is prepared by reacting, 1,1-dimethyl-3-(4-chlorocarbonylphenyl)-propylamine with excess ammonia. The chlorocarbonylphenyl compound may be prepared, for example, by reacting 1,1-dimethyl-1-hydroxy-3-(4-hydroxycarbonylphenyl)propane with sodium cyanide, hydrolysing the N-formyl amine so obtained, salifying and reacting the salt with thionyl chloride.

The compounds of formula (I) are useful in potentiating the antineoplastic effects of oncolytic drugs. Alkylation of the 1,1-dimethyl-3-(4-aminocarbonylphenyl)propylamine of the invention with a haloacetophenone, i.e. a benzoylmethyl halide provides an N-benzolymethyl-1,1-dimethyl-3-aryl-propylamine. The benzoyl carbonyl moiety is then reduced to provide a compound of formula (I) in which $R^2$ is an aminocarbonyl group.

In other words, a carbonyl compound of formula (II):

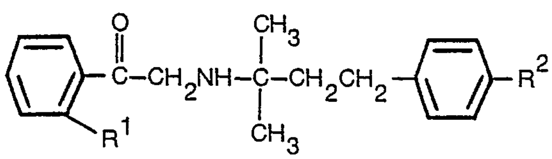

(II)

is reduced. The compounds of formula (II) are novel, and provided in one aspect of the invention of our copending application is the single stage process for producing compounds of formula (I) by reduction of compounds of formula (II). The reduction is preferably effected within the temperature range from 15 to 115°C. in an inert organic solvent.

As an illustration of such generalized synthetic process, a benzoylmethyl halide such as benzoylmethyl bromide, *o*-fluorobenzoylmethyl iodide, or the like, is reacted with a propylamine derivative such as 1,1-dimethyl-3-(4-aminocarbonylphenyl)propylamine. The benzoylmethyl halide and the 1,1-dimethyl-3-aryl-propylamine, in particular 1,1-dimethyl-3-(4-aminocarbonylphenyl)propylamine, are generally utilized in approximately equimolar quantities; however, an excess of the amine can be utilized if desired and then later separated from the desired product by routine methods. The alkylation reaction is best carried out in a suitable solvent such as diethyl ether, benzene, dichloromethane, or the like, and normally is complete within about two to ten hours when carried out at about 25 to about 70°C. The N-benzoylmethyl-1,1-dimethyl-3-arylpropylamine, particularly the (4-aminocarbonylphenyl)propylamine, which is formed normally is recovered simply by filtering the reaction mixture, thereby removing any unreacted starting materials, and then removing the solvent from the filtrate, for instance by evaporating. The product thus formed can be further purified if desired by routine methods such as salt formation and crystallization. The N-benzoylmethyl-(4-aminocarbonylphenyl)propylamine derivative next is reduced to provide a compound of formula (I). Such reduction is accomplished by any of the commonly used methods to reduce a carbonyl group to a carbinol moiety, including catalytic hydrogenation utilizing catalysts such as platinum or palladium, or metal hydride reductions utilizing agents such as sodium borohydride and the like.

Another and often preferred method for preparing compounds of formula (I) comprises reacting a 1,1-dimethyl 3-(4-substituted phenyl)propylamine particularly a (4-aminocarbonylphenyl)propylamine with styrene oxide, *o*-fluorostyrene oxide, or even an optically active styrene oxide. The amine and styrene oxide are utilized in approximately equimolar quantities, and the reaction is carried out in a polar organic solvent such as methanol or ethanol. The condensation to provide a compound of formula (I) generally is complete within about ten to twenty hours when the reaction is carried out at about 50 to about 80°C., although temperatures from 20 to 150°C. can be utilized. The product is isolated by simply removing the reaction solvent, for instance by evaporation under reduced pressure. The product amine can be further purified if desired by crystallization from solvents such as acetone or ethyl acetate, or alternatively can be converted to a salt and crystallized from solvents such as methanol or isopropanol.

Representative of the compounds of formula I are

R-N-(2-phenyl-2-hydroxyethyl)-1,1-dimethyl-3-(4-aminocarbonylphenyl)propylamine;

R-N-(2-phenyl-2-hydroxyethyl)-1,1-dimethyl-3-(4-aminocabonylphenyl)propylaminium *p*-toluenesulfonate; and the like.

The compounds of formula (I) cause a significant and unexpected increase in the effectiveness of currently used oncolytic agents. Such a surprising result permits the use of now clinically used antineoplastic drugs in lesser amounts than now permitted for effective treatment, or permits more effective treatment when administering oncolytic drugs at their current clinical levels. Since the compounds of formula (I) are neither antineoplastic in themselves nor toxic to healthy cells at contemplated doses, the overall effect of combination chemotherapy according to this invention is that greater efficacy and fewer toxic side effects from the use of known oncolytic drugs can be realized in the treatment of tumors.

In an effort to more fully describe specific aspects of the invention, the following detailed preparation and Examples are provided. The Examples are by no means intended to limit the invention and should not be so construed.

Preparation 1

1,1-Dimethyl-3-(4-methoxyphenyl)propylamine

A cold solution of 56 g. of sodium cyanide in 125 ml. of acetic acid was stirred and diluted by the addition of a solution of 140 ml. of conc. sulfuric acid containing 125 ml. of acetic acid. The the stirred acid solution was addded 170.8 g. of 1,1-dimethyl-1-hydroxy-3-(4-methoxyphenyl)propane portion-wise over ten minutes. Following complete addition of the alcohol, the reaction mixture was heated to 75°C. for thirty minutes and then cooled to 25°C. and stirred for an additional two hours. The reaction mixture next was poured into 400 ml. of ice water and made neutral by the addition of sodium carbonate. The aqueous solution was extracted several times with diethyl ether, and the ethereal extracts were combined, washed with water and dried. Removal of the solvent by evaporation under reducded pressure provided 142.8 g. of N-formyl-1,1-dimethyl-3-(4-methoxyphenyl)propylamine.

The latter compound was dissolved in 1000 ml. of 2.7 N hydrochloric acid and the acid reaction mixture was heated at 100°C. for twelve hours. After cooling the acidic reaction mixture, it was added to 500 ml. of water, and then washed with ethyl acetate. The aqueous layer was made alkaline by the addition of sodium hydroxide, and the alkaline reaction mixture was extracted with diethyl ether. The ethereal extracts were combined, washed with water and dried. Evaporation of the solvent and distillation of the product afforded 72.9 g. of 1,1-dimethyl-3-(4-methoxyphenyl)propylamine. B.P. 110—120°C at 133.3 $N/M^2$ (1.0 torr).

Example 1

Following the procedure set forth in Preparation 1, 1,1-dimethyl-1-hydroxy-3-(4-hydroxycarbonylphenyl)propane was reacted with sodium cyanide to provide, after hydrolysis of the intermediate N-formyl amine, 1,1-dimethyl-3-(4-hydroxycarbonylphenyl)propylamine. The amine thus formed was converted to the hydrochloride salt and the salt was reacted with thionyl chloride to provide 1,1-dimethyl-3-(4-chlorocarbonylphenyl)propylamine. The acid chloride so produced was reacted with an excess of ammonia to provide 1,1-dimethyl-3-(4-aminocarbonylphenyl)propylamine.

Example 2

N-(2-Phenyl-2-hydroxyethyl)-1,1-dimethyl-3-(4-aminocarbonylphenyl)propylamine

A solution of 3.9 g. of 1,1-dimethyl-3-(4-aminocarbonylphenyl)propylamine in 150 ml. of ethanol containing 2.8 g of styrene oxide was heated to reflux and stirred for twelve hours. After cooling the reaction mixture to room temperature, the solvent was removed therefrom by evaporation to provide a solid residue. The solid was crystallized from 100 ml. of hot diethyl ether to provide 3.3 g. of crystalline product. The product thus formed was recrystallized twice from hot acetone to afford 1.98 g. of N-(2-phenyl-2-hydroxyethyl)-1,1-dimethyl-3-(4-aminocarbonylphenyl)propylamine. M.P. 146—150°C.

Analysis calc. for $C_{20}H_{26}N_2O_2$

| | | | |
|---|---|---|---|
| Theory: | C, 73.59; | H, 8.03; | N, 8.58. |
| Found: | C, 73.40; | H, 7.91; | N, 8.33. |

The amine base thus formed was dissolved in methanol and added to a solution of hydrogen chloride in diethyl ether. The solid precipitate which formed was collected and recrystallized from 30 ml. of acetone and 5 ml. of ethanol to provide 1.79 g. of N-(2-phenyl-2-hydroxyethyl)-1,1-dimethyl-3-(4-amino-carbonylphenyl)propylaminium chloride. M.P. 204—206°C.

Analysis calc. for $C_{20}H_{27}N_2N_2O_2Cl$

| | | | | |
|---|---|---|---|---|
| Theory: | C, 66.19; | H, 7.50; | N, 7.72; | Cl, 9.77. |
| Found: | C, 66.05; | H, 7,42; | N, 7.53; | Cl, 10.06. |

Example 3

Following the procedure set forth in Example 2, 4.6 g. of *o*-fluorostyrene oxide was reacted with 5.8 g. of 1,1-dimethyl-3-(4-aminocarbonylphenyl)propylamine to provide, after crystallization from methanol and ethyl acetate, 2.0 g of N-[2-(2-fluorophenyl)-2-hydroxyethyl]-1,1-dimethyl-3-(4-aminocarbonylphenyl)propylamine.

Analysis calc. for $C_{20}H_{25}N_2O_2F$

| | | | |
|---|---|---|---|
| Theory: | C, 69.74; | H, 7.32; | N, 8.13. |
| Found: | C, 69.58; | H, 7.12; | N, 8.28. |

The amine base thus formed was converted to its hydrochloride salt by reaction with hydrogen choride in diethyl ether to give 1.2 g. of N-[2-(2-fluorophenyl)-2-hydroxyethyl]-1,1-dimethyl-3-(4-aminocarbonylphenyl)propylaminium chloride. M.P. 227—230°C.

Analysis calc. for $C_{20}H_{26}N_2O_2ClF$

| | | | |
|---|---|---|---|
| Theory: | C, 63.07; | H, 6.88; | N, 7.35. |
| Found: | C, 63.21; | H, 7.12; | N, 7.45. |

Example 4

By following the general procedure set out in Example 2, 1,1-dimethyl-3-(4-aminocarbonylphenyl)propylamine was reacted with optically active R-*o*-fluorostyrene oxide to provide R-N-[2-(2-fluorophenyl)-2-hydroxyethyl]-1,1-dimethyl-3-(4-aminocarbonylphenyl)propylamine.

Example 5

The following formulation was prepared for use in the treatment of solid tumors.

| | |
|---|---|
| N-(2-phenyl-2-hydroxyethyl)-1,1-dimethyl-3-(4-aminocarbonylphenyl)-propylaminium chloride | 100 mg. |
| starch | 300 mg. |
| lactose | 100 mg. |
| | 500 mg. |

The above ingredients are thoroughly mixed and the mixture is lubricated with 1% magnesium stearate and compressed into tablets. Such tablets are administered at the rate of about 1 to 2 tablets per day for a patient weighing about 60 kg. in combination with an effective dose of cytoxan at about 2 mg./kg. administered orally once daily for the therapy of solid neoplasms such as neuroblastoma and adenocarcinoma of the ovary.

Example 6

Formulation of intravenous administration

| | |
|---|---|
| R-N-[2-(2-fluorophenyl)-2-hydroxyethyl]-1,1-dimethyl-3-(4-aminocarbonylphenyl)-propylamine | 250 mg. |
| Isotonic saline | 50 ml. |

The above solution is administered once or twice a day in combination with a normal dosage regimen of an agent such as 6-mercaptopurine to subject weighing from about 50 to about 70 kg.

Neoplasms to be treated with the formulation include solid tumors of the breast, colon, stomach, pancreas, ovary, urinary bladder and the like.

**Claims**

1. The compound 1,1-dimethyl-3-(4-aminocarbonylphenyl)propylamine.

2. A method of preparing the compound of claim 1, which comprises reacting 1,1-dimethyl-3-(4-chlorocarbonylphenyl)propylamine with excess ammonia.

3. A method according to claim 2 in which the 1,1-dimethyl-3-(4-chlorocarbonylphenyl)-propylamine is prepared by a method which comprises reaction 1,1-dimethyl-1-hydroxy-3-(4-hydroxy-carbonylphenyl)propane with sodium cyanide, hydrolysing the N-formyl amine, salifying and reacting the salt with thionyl chloride.

**Revendications**

1. Composé, à savoir la 1,1-diméthyl-3-(4-aminocarbonylphényl)propylamine.

2. Procédé de préparation du composé suivant la revendication 1, caractérisé en ce qu'il consiste à faire réagir la 1,1-diméthyl-3-(4-chlorocarbonylphényl)propylamine avec de l'ammoniac en excès.

3. Procédé suivant la revendication 2, caractérisé en ce qu'on préparé la 1,1-diméthyl-3-(4-chlorocarbonylphényl)propylamine par un procédé consistant à faire réagir le 1,1-diméthyl-1-hydroxy-3-(4-hydroxycarbonylphényl)propane avec le cyanure de sodium, hydrolyser la N-formyl-amine, salifier et faire réagir le sel avec du chlorure de thionyle.

**Patentansprüche**

1. 1,1-Dimethyl-3-(4-aminocarbonylphenyl)propylamin.

2. Verfahren zur Herstellung der Verbindung von Anspruch 1, dadurch gekennzeichnet, daß man 1,1-Dimethyl-3-(4-chlorocarbonylphenyl)propylamin mit überschüssigem Ammoniak umsetzt.

3. Verfahren nach Anspruch 2, dadurch gekennzeichnet, daß man das 1,1-Dimethyl-3-(4-chlorocarbonylphenyl)propylamin herstellt durch Umsetzung von 1,1-Dimethyl-1-hydroxy-3-(4-hydroxy-carbonylphenyl)propan mit Natriumcyanid, Hydrolyse des N-Formylamins, Salzbildung und Umsetzung des Salzes mit Thionylchlorid.